# EUROPEAN PATENT APPLICATION

(11) **EP 2 620 424 A1**
(43) Date of publication of application: **31.07.2013**
(21) Application number: 12305091.6
(22) Date of filing: 25.01.2012
(51) Int. Cl.: C07C 37/00, C07C 39/11

(54) **Method of preparation of hydroxytyrosol**

(71) Applicant: Université de Bordeaux I, 33400 Talence (FR); Centre National de la Recherche Scientifique (C.N.R.S), 75016 Paris (FR); Societe Civile Immobiliere Girondina, 33500 Lalande de Pomerol (FR)
(72) Inventor: The designation of the inventor has not yet been filed
(74) Representative: Jacobson, Claude

(57) **Abstract**

The present invention relates to a method of preparation of hydroxytyrosol from eugenol, said method comprising:
- a step of oxidative cleavage, in particular in the presence of an oxidative reactant such as ozone followed by the addition in situ of a mild hydride source such as NaBH₄, and
- a step of demethylation, in particular in the presence of a Lewis acid, such as All₃, or of an inorganic hypervalent iodine compound, such as NalO₄, followed by a reductive treatment.

## Description

The present invention relates to a method or preparation of hydroxytyrosol from a naturally occurring and commercially available starting material.

Hydroxytyrosol (2-(3,4-dihydroxyphenyl)ethanol) is one of the major phenolic compounds present in the olive fruit and olive oil, and in the vegetation waters deriving from its production. It is a naturally simple plant-derived catecholic molecule that exhibits a strong antioxidant activity. It may be used as an antioxidant in the pharmaceutical field, for example for the prevention and/or the treatment of cardiovascular diseases, in the cosmetic field, in the food field and in the agrochemical field.

However, further consideration of the application potential of hydroxytyrosol as an alternative non-toxic food preservative with additional therapeutically-relevant properties has been thwarted by the lack of large quantities of hydroxytyrosol at low cost. Indeed, hydroxytyrosol is only commercially available at high price. It is also present as component in various vegetal extracts, but in low concentration.

Because of its presence in the vegetal matrixes of natural source, the only industrial source of hydroxytyrosol so far remains its recovery from olive oil by-products.

These low-yield methods of recovery are often laborious and require the use of considerable amount of organic harmful and inflammable solvents and suitable equipments of extraction and purification. At best, 4 to 5 kg of crude hydroxytyrosol can be obtained from 1 000 kg of "alperujo", a by-product of modern olive oil mill processing.

A few chemical syntheses of hydroxytyrosol have been reported, but they rely on relatively expensive starting materials, and/or are low-yielding. These methods utilize expensive starting materials such as 3,4-dimethoxyphenyl ethanol, 3,4-dimethoxyphenyl acetic acid, 3,4-dimethoxy benzaldehyde, (3,4-dihydroxyphenyl)acetic acid or its methyl ester, and require the use of hazardous reagents, such as lithium aluminium hydride (LiAlH₄), and anhydrous solvents, such as tetrahydrofuran.

At this date, according to what is reported so far with respect to industrial applications of hydroxytyrosol, there is a lack of an economic chemical synthesis method that could be easily scaled-up. Such method, starting from a material commercially available at a low cost or obtainable from natural sources, would make it possible the preparation of hydroxytyrosol through few steps.

The present invention aims at providing a convenient and sustainable method of preparation of hydroxytyrosol using a renewable and available starting material.

The present invention also aims at providing a method suitable for an industrial-scale production of hydroxytyrosol.

According to one embodiment, the present invention relates to a method for preparing hydroxytyrosol (1) 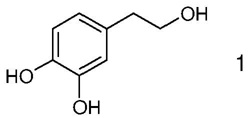 from eugenol (2) 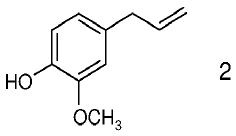 comprising a step of oxidative cleavage.

Naturally occurring eugenol (4-allyl-2-methoxyphenol) is the principal aromatic constituent of clove oil (approximately 80% w/w), extracted by steam distillation or extraction from leaves, bud and stem of clove trees belonging to the family *Myrtacea.* Eugenol is commercially available in large quantities, making it an economically realistic feedstock. So far, eugenol was widely used in dentistry, as flavoring agent in cosmetic and food products, and as a key ingredient in Indonesian kretek cigarettes.

From this inexpensive ortho-oxygenated starting phenylpropanoid, a direct access to hydroxytyrosol has been found, which does not implement large quantities of solvents.

The term "oxidative cleavage" refers to the cleavage of a C=C alkene double bond and the formation of C-O bonds from the carbon atoms originally forming said double bond. The oxidative cleavage is carried out in the presence of an oxidative reagent.

The term "oxidative reagent" also refers to oxidising reagents, oxidants and oxidisers.

According to one embodiment, the step of oxidative cleavage corresponds to the cleavage of the allylic side-chain of eugenol (or a compound derived thereof, such as 4-allyl 2-hydroxyphenol) into a hydroxyethyl unit, corresponding to the hydroxyethyl side-chain of hydroxytyrosol, according to the following scheme:

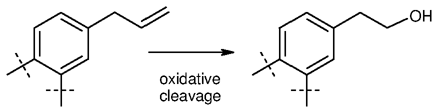

According to one embodiment, the step of oxidative cleavage is carried out in the presence of an oxidative reagent selected from the group consisting of O₃, KMnO₄, NalO₄, OsO₄/NalO₄, iodosylbenzene (PhlO)/aqueous HBF₄, (diacetoxy)iodosylbenzene [Phl(OAc)₂]/OsO₄ and iodobenzene/potassium peroxymonosulfate.

Oxone® may be used as a source of potassium peroxymonosulfate.

The use of Phl(OAc)₂/OsO₄ as oxidative reagent is notably described in Nicolaou et al., Org. Lett. (2010), 12 (7), p.1552-1555.

The use of PhlO/aqueous HBF₄ as oxidative reagent is notably described in Ochiai et al., J. Am. Chem. Soc. (2007), vol.129, N° 10, p.2772-2772.The step of oxidative cleavage is for example carried out in the presence of O₃.

The step of oxidative cleavage corresponds for example to an ozonolysis reaction, by which the allylic side-chain of eugenol (or a compound derived thereof) is transformed into a hydroxyethyl unit, corresponding to the hydroxyethyl side-chain of hydroxytyrosol.

According to one embodiment, the step of oxidative cleavage is carried out in a solvent.

According to one embodiment, the step of oxidative cleavage is carried out in a polar solvent.

As solvent suitable for the step of oxidative cleavage, one may cite lower alcohol solvents, for example methanol and ethanol, preferably ethanol.

According to one embodiment, the step of oxidative cleavage is carried out at a temperature comprised from -78°C to 100°C, preferably at low temperature, for example from -78°C to 0°C, advantageously from -78°C to -30°C.

According to this embodiment, the step of oxidative cleavage comprises a reductive treatment, preferably in the presence of a mild source of hydride ions, such as NaBH₄.

The term "hydride" refers to a compound containing metal or metalloid bonds to hydrogen, having reducing properties, such as borohydride and aluminium hydride.

The reductive treatment may be carried out in the presence of a reductive reagent, such as NaBH₄ and BH₃-DMS (dimethylsulfide).

According to this embodiment, the step of oxidative cleavage is the cleavage of the allylic side-chain of eugenol (or a compound derived thereof, such as 4-allyl 2-hydroxyphenol) into an ozonide unit, followed by the reductive treatment of said ozonide intermediate to give hydroxyethyl side-chain of hydroxytyrosol, according to the following scheme:

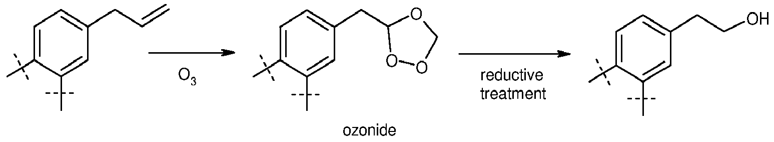

The reductive treatment is for example an *in situ* hydride reduction, carried out by addition to the reaction mixture of a hydride such as NaBH₄.

When the step of oxidative cleavage is an ozonolysis reaction carried out in the presence of ozone O₃, the reaction may be carried out in ethanol or methanol, preferably in ethanol.

When the step of oxidative cleavage is an ozonolysis reaction carried out in the presence of O₃, the reaction may be carried out at low temperature, for example comprised from -78°C to -30°C.

According to another embodiment, the method of the invention comprises a step of demethylation.

The term "demethylation" refers to the reaction of transformation of a -OCH₃ function into a -OH function, via the cleavage of a O-C bond and the formation of a O-H bond.

According to one embodiment, the step of demethylation corresponds to the transformation of the methoxy function of eugenol (or a compound derived thereof, such as homovanillyl alcohol) into a -OH function, corresponding to one of the two phenol functions of hydroxytyrosol, according to the following scheme:

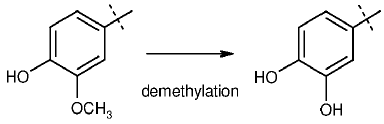

According to one embodiment, the step of demethylation is carried out in the presence of a reagent selected from the group consisting of:
- Lewis acids, such as All₃, BBr₃, LiCl, AlCl₃-EtSH and AlBr₃-EtSH, and
- hypervalent iodine oxidants, such as NalO₄, Dess-Martin periodinane DMP, IBX and SIBX.

According to one embodiment, the step of demethylation is for example carried out in the presence of a Lewis acid, or a mixture of Lewis acids.

As suitable Lewis acids according to this embodiment, one may cite, inter aliae, H⁺, BH₃, B₂H₆, BF₃.Et₂O, BBr₃, Al₂Cl₆, AlCl₃, AlCl₃-EtSH, AlBr₃-EtSH, All₃, AlF₃, SiF₄, PCl₅, SF₄ and LiCl.

According to this embodiment, the step of demethylation may be carried out in the presence of All₃.

The reagent All₃ may be formed *in situ* by mixing aluminium powder and iodine in a refluxing solvent.

A catalytic amount of a catalyst may be added to the reaction mixture during the step of demethylation, for example a phase transfer catalyst such as tetrabutylammonium iodide (TBAI) or tetrabutylammonium bromide (TBAB).

According to this embodiment, the step of demethylation is carried out in a solvent.

As solvent suitable according to this embodiment, one may cite benzene, toluene, cyclohexane, acetonitrile, ethyl acetate, dimethylformamide and dichloromethane, preferably cyclohexane.

According to this embodiment, the step of demethylation is carried out at a temperature comprised from -78°C to 100°C, preferably at room temperature or higher, for example from room temperature to 100°C.

According to this embodiment, the step of demethylation is carried out at the refluxing temperature of the solvent.

According to another embodiment, the step of demethylation is carried out in the presence of a hypervalent iodine oxidant.

As suitable hypervalent iodine oxidant according to this embodiment, one may cite: NalO₄, DMP (Dess-Martin periodinane), IBX (2-iodoxy benzoic acid) and SIBX (stabilized IBX).

According to this embodiment, the step of demethylation may be carried out in the presence of NalO₄.

According to this embodiment, the step of demethylation is carried out in a solvent, preferably in a polar solvent.

As solvent suitable according to this embodiment, one may cite ethyl acetate, methanol, ethanol and tetrahydrofuran.

According to this embodiment, the step of demethylation is carried out at a temperature comprised from -78°C to 100°C, preferably at room temperature.

According to one embodiment, the step of oxidative cleavage is carried out prior to the step of demethylation.

According to this embodiment, the method of the invention comprises a step of oxidative cleavage of eugenol (2) to obtain homovanillyl alcohol (3): 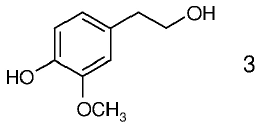 wherein said step is carried out in the presence of an oxidative reagent selected from the group consisting of O₃, KMnO₄, NalO₄, OsO₄/NalO₄, iodosylbenzene (PhlO)/aqueous HBF₄, (diacetoxy)iodosylbenzene [Phl(OAc)₂]/OsO₄ and iodobenzene/potassium peroxymonosulfate.

According to this embodiment, the method of the invention comprises a step of demethylation of homovanillyl alcohol (3) to obtain hydroxytyrosol (1),
wherein said step is carried out in the presence of a reagent selected from the group consisting of:
- Lewis acids, such as All₃, BBr₃, LiCl, AlCl₃-EtSH and AlBr₃-EtSH, and
- hypervalent iodine oxidants, such as NalO₄, Dess-Martin periodinane (DMP), IBX and SIBX.

According to one embodiment, the present invention relates to a method for preparing hydroxytyrosol from eugenol, comprising:
- a step of oxidative cleavage of eugenol to obtain (3), wherein said step is carried out in the presence of an oxidative reagent selected from the group consisting of O₃, KMnO₄, NalO₄, OsO₄/NalO₄, iodosylbenzene (PhlO)/aqueous HBF₄, (diacetoxy)iodosylbenzene [Phl(OAc)₂]/OsO₄ and iodobenzene/potassium peroxymonosulfate, and
- a step of demethylation of (3) to obtain hydroxytyrosol, wherein said step is carried out in the presence of a reagent selected from the group consisting of:
- Lewis acids, such as All₃, BBr₃, LiCl, AlCl₃-EtSH and AlBr₃-EtSH, and
- hypervalent iodine oxidants, such as NalO₄, Dess-Martin periodinane DMP, IBX and SIBX.

According to another embodiment, the step of demethylation is carried out prior to the step of oxidative cleavage.

According to this embodiment, the method of the invention comprises a step of demethylation of eugenol (2) to obtain 4-allyl 2-hydroxyphenol (4): 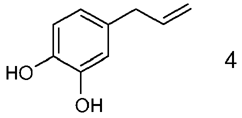 wherein said step is carried out in the presence of a reagent selected from the group consisting of:
- Lewis acids, such as All₃, BBr₃, LiCl, AlCl₃-EtSH and AlBr₃-EtSH, and
- hypervalent iodine oxidants, such as NalO₄, Dess-Martin periodinane DMP, IBX and SIBX.

According to this embodiment, the method of the invention comprises a step of oxidative cleavage of 4-allyl 2-hydroxyphenol (4) to obtain hydroxytyrosol (1),
wherein said step is carried out in the presence of an oxidative reagent selected from the group consisting of O₃, KMno₄, Nalo₄, Oso₄/NalO₄, iodosylbenzene (PhlO)/aqueous HBF₄, (diacetoxy)iodosylbenzene [Phl(OAc)₂]/OsO₄ and iodobenzene/potassium peroxymonosulfate.

According to another embodiment, the method of the invention comprises:
- a step of demethylation of eugenol to obtain (4), wherein said step is carried out in the presence of a reagent selected from the group consisting of:
- Lewis acids, such as All₃, BBr₃, LiCl, AlCl₃-EtSH and AlBr₃-EtSH, and
- hypervalent iodine oxidants, such as NalO₄, Dess-Martin periodinane DMP, IBX and SIBX,
   and
- a step of oxidative cleavage of (4) to obtain hydroxytyrosol, wherein said step is carried out in the presence of an oxidative reagent selected from the group consisting of O₃, KMnO₄, NalO₄, OsO₄/NalO₄, iodosylbenzene (PhlO)/aqueous HBF₄, (diacetoxy)iodosylbenzene [Phl(OAc)₂]/OsO₄ and iodobenzene/potassium peroxymonosulfate.

### General Experimental Procedures

All moisture and oxygen sensitive reactions were carried out in flame-dried glassware under inert atmosphere with dry solvents. Methanol (MeOH), ethyl acetate (EtOAc), light petroleum diethylic ether (Et₂O), chloroform (CHCl₃), acetonitrile, ethanol and cyclohexane were purchased from Acros and used without prior purification. Evaporations were conducted under reduced pressure at temperatures less than 40°C. Further drying of the residues was accomplished under high vacuum. Reactants including eugenol were all purchased from Aldrich and used as received. Reactions were monitored by thin layer chromatography (TLC) carried out on 0.25 mm E. Merck silica plates (60 F₂₅₄) using UV light for visualization and a phosphomolybdic acid solution and heat as the developing agent. Column chromatography was carried out under positive pressure using 40-63 µm silica gel (Merck) and the indicated solvents. IR spectra were recorded on a Perkin Elmer Targon 1000 spectrometer. NMR spectra of samples in the indicated solvent were run at 300 MHz unless otherwise noted and calibrated using residual solvent as an internal standard. Carbon multiplicities were determined by DEPT 135 experiments. Data processings were carried out with Topspin® Bruker software version 2.1. Electron impact and liquid secondary ion mass spectrometry low resolution mass spectrometric analyses (EIMS) were obtained from the mass spectrometry laboratory at the CESAMO, Bordeaux 1 University.

### Example 1: Preparation of homovanillyl alcohol (3) from eugenol (2)

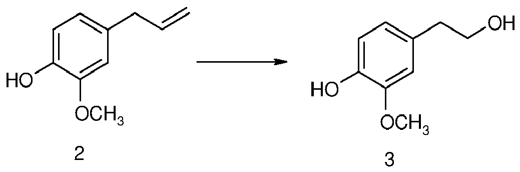

Eugenol (2) (20.0 g, 122 mmol) was dissolved in ethanol (200 mL) and ozonolyzed at -55°C. The cooling bath was removed as soon as TLC monitoring, eluting with light petroleum/EtOAc (1:1), indicated the complete disappearance of eugenol (2), and the reaction mixture was then slowly quenched by adding NaBH₄ (6.03 g, 158 mmol) in small portions. This mixture was stirred at room temperature overnight, after which time it was carefully acidified with aqueous 10% HCl, concentrated under reduced pressure and extracted three times with Et₂O. The combined organic extracts were washed three times with brine, dried over Na₂SO₄, filtered and evaporated to give a residue, which was further dried under high vacuum overnight to give homovanillyl alcohol (3) (20.1 g, 98%) as a dark yellow oil. IR (NaCl) 3466 cm⁻¹; ¹H NMR (CDCl₃) δ 2.77 (t, *J* = 6.4 Hz, 2H), 3.80 (t, *J* = 6.4 Hz, 2H), 3.84 (s, 3H), 6.68-6.84 (m, 3H), ¹³C NMR (CDCl₃, 62.9 MHz) δ 148.8, 147.5, 130.9, 120.8, 112.1, 111.3, 63.6, 55.7, 38.6; EIMS *m*/*z* (rel. intensity) 168 (M⁺, 21), 150 (12), 137 (100), 122 (16), 94 (20), 77 (18). This oil was used without further purification.

### Example 2: Preparation of hydroxytyrosol (1) from homovanillyl alcohol (3)

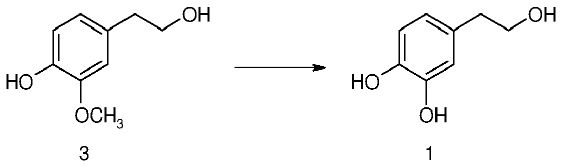

### Method A

A stirring mixture of aluminum powder (2.2 g, 81.5 mmol) and iodine (20.9 g, 164.6 mmol) in acetonitrile (100 mL) was refluxed until the brown color of the mixture turned to green (*ca.* 2 h). The mixture was then cooled to room temperature and a solution of homovanillyl alcohol (3) (4.68 g, 27.9 mmol) and TBAI (0.4 g, 1.08 mmol) in acetonitrile (25 mL) was added dropwise. The resulting mixture was refluxed for 50 min, then cooled in an ice/water bath, hydrolyzed with water (100 mL) and concentrated under reduced pressure. The concentrate was diluted in EtOAc (50 mL) and washed with aqueous 10% HCl (50 mL). After separation, the aqueous phase was extracted several times with EtOAc (5 x 50 mL) and the combined organic layers were washed with brine (50 mL), dried with Na₂SO₄, filtered and evaporated. The residue was submitted to column chromatography, eluting with CHCl₃/MeOH (7:1), to furnish hydroxytyrosol (1) as a yellowish oil (2.3, 54 %).

### Method B

A solution of homovanillyl alcohol (3) (0.980 g, 5.82 mmol) in 40 mL of ethyl acetate was introduced in a separatory funnel (100 mL) and was treated with a 20 mL of an aqueous saturated solution of NalO₄ (2.56 g, 11,98 mmol). A dark red coloration of the resulting solution was immediately observed. After shaking and quick decantation, 9.5 mL of the aqueous phase was collected and then extracted with EtOAc (3x20 mL). The combined organic phases were further washed with 3 mL of water in order to remove possible residual iodine products. The washed mixture was then treated with 8 mL of a saturated aqueous solution of Na₂S₂O₄, (2.23 g, 12.81 mmol). Discharge of the reaction mixture's color was observed upon shaking the reaction mixture. The organic phase was then washed with 4 mL of brine, dried over Na₂SO₄, filtered, and evaporated leading to hydroxytyrosol (1) as a yellowish oil (0.71 mg, 78 %). IR (NaCl) 3508 cm⁻¹; ¹H NMR (CD₃OD) δ 2.66 (t, *J* = 6.4 Hz, 2H), 3.67 (t, *J* = 6.7 Hz, 2H), 4.90 (br, 3H), 6.50-6.69 (m, 3H); 13C NMR (CD₃OD, 62.9 MHz) δ 146.0, 144.6, 131.7, 121.2, 117.0, 116.3, 64.6, 39.6; EIMS *m*/*z* (rel. intensity) 154 (M⁺, 35), 123 (100), 77 (20), 51 (14); UV (methanol) (Iₘₐₓ = 218, e = 4975; Iₘₐₓ = 282, e = 2477). This oil partially crystallized upon storage in the freezer at -20°C, but the resulting yellowish broad needles rapidly melted upon warming up to room temperature.

## Claims

1. A method for preparing hydroxytyrosol (1): 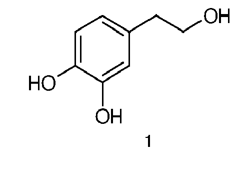 from eugenol (2): 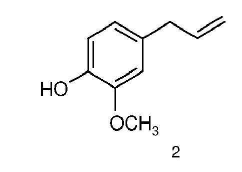 comprising a step of oxidative cleavage.

2. The method according to claim 1, wherein the step of oxidative cleavage is carried out in the presence of an oxidative reagent selected from the group consisting of O₃, KMnO₄, NalO₄, OsO₄/NalO₄, iodosylbenzene (PhlO)/aqueous HBF₄, (diacetoxy)iodosylbenzene [Phl(OAc)₂]/OsO₄ and iodobenzene/potassium peroxymonosulfate.

3. The method according to claim 2, wherein the step of oxidative cleavage comprises a reductive treatment, preferably in the presence of an hydride, such as NaBH₄.

4. The method according to any one of claims 1 to 3, comprising a step of demethylation.

5. The method according to claim 4, wherein the step of demethylation is carried out in the presence of a reagent selected from the group consisting of:
- Lewis acids, such as All₃, BBr₃, LiCl, AlCl₃-EtSH and AlBr₃-EtSH, and
- hypervalent iodine oxidants, such as NalO₄, Dess-Martin periodinane (DMP), IBX and SIBX.

6. The method according to claim 4, wherein the step of oxidative cleavage is carried out prior to the step of demethylation.

7. The method according to claim 6, comprising:
a step of oxidative cleavage of eugenol (2) 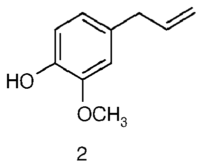
to obtain homovanillyl alcohol (3) 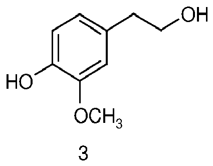
wherein said step is carried out in the presence of an oxidative reagent selected from the group consisting of O₃, KMnO₄, NalO₄, OsO₄/NalO₄, iodosylbenzene (PhlO)/aqueous HBF₄, (diacetoxy)iodosylbenzene [Phl(OAc)₂]/OsO₄ and iodobenzene/potassium peroxymonosulfate.

8. The method according to claim 7, comprising:
a step of demethylation of homovanillyl alcohol (3) 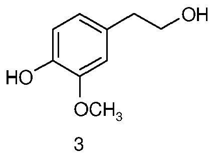
to obtain hydroxytyrosol (1) 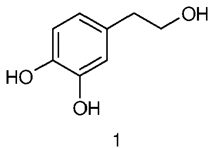
wherein said step is carried out in the presence of a reagent selected from the group consisting of:
- Lewis acids, such as All₃, BBr₃, LiCl, AlCl₃-EtSH and AlBr₃-EtSH, and
- hypervalent iodine oxidants, such as NalO₄, Dess-Martin periodinane (DMP), IBX and SIBX.

9. The method according to claim 4, wherein the step of demethylation is carried out prior to the step of oxidative cleavage.

10. The method according to claim 9, comprising:
a step of demethylation of eugenol (2) 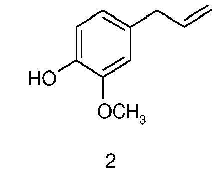
to obtain 4-allyl 2-hydroxyphenol (4) 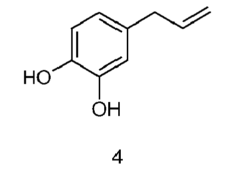
wherein said step is carried out in the presence of a reagent selected from the group consisting of:
- Lewis acids, such as All₃, BBr₃, LiCl, AlCl₃-EtSH and AlBr₃-EtSH, and
- hypervalent iodine oxidants, such as NalO₄, Dess-Martin periodinane DMP, IBX and SIBX.

11. The method according to claim 10, comprising:
a step of oxidative cleavage of 4-allyl 2-hydroxyphenol (4) 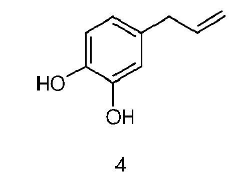 to obtain hydroxytyrosol (1) 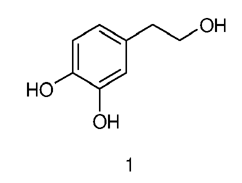
wherein said step is carried out in the presence of an oxidative reagent selected from the group consisting of O₃, KMnO₄, NalO₄, OsO₄/NalO₄, iodosylbenzene (PhlO)/aqueous HBF₄, (diacetoxy)iodosylbenzene [Phl(OAc)₂]/OsO₄ and iodobenzene/potassium peroxymonosulfate.
